# EUROPEAN PATENT APPLICATION

(11) **EP 4 289 831 A1**
(43) Date of publication of application: **13.12.2023**
(21) Application number: 22749860.7
(22) Date of filing: 08.02.2022
(51) Int. Cl.: C07D 311/78, G02B 1/04, G02B 5/23, G02C 7/10

(54) **PHOTOCHROMIC COMPOUND, PHOTOCHROMIC COMPOSITION, PHOTOCHROMIC ARTICLE AND EYEGLASSES**

(30) Priority: 08.02.2021 JP 2021018608
(71) Applicant: Hoya Lens Thailand Ltd., Pathumthani 12130 (TH)
(72) Inventor: KAWAKAMI Hironori, Tokyo 160-8347 (JP); KOBAYASHI Kei, Tokyo 160-8347 (JP); MATSUE Aoi, Tokyo 160-8347 (JP)
(74) Representative: Hoffmann Eitle
(86) International application number: PCT/JP2022/004931
(87) International publication number: WO 2022/168989

(57) **Abstract**

Provided are photochromic compounds represented by General Formula 1, photochromic compounds represented by General Formula 2 and photochromic compounds represented by General Formula 3. In General Formula 1, R¹⁰⁰ to R¹¹¹, and X¹ and X² each independently represent a hydrogen atom or a substituent, and two or more substituents may be bonded to form a ring structure. In General Formula 2, R²⁰⁰ to R²¹¹, and X³ and X⁴ each independently represent a hydrogen atom or a substituent, and two or more substituents may be bonded to form a ring structure. In General Formula 3, R³⁰⁰ to R³¹¹, and X⁵ and X⁶ each independently represent a hydrogen atom or a substituent, and two or more substituents may be bonded to form a ring structure.

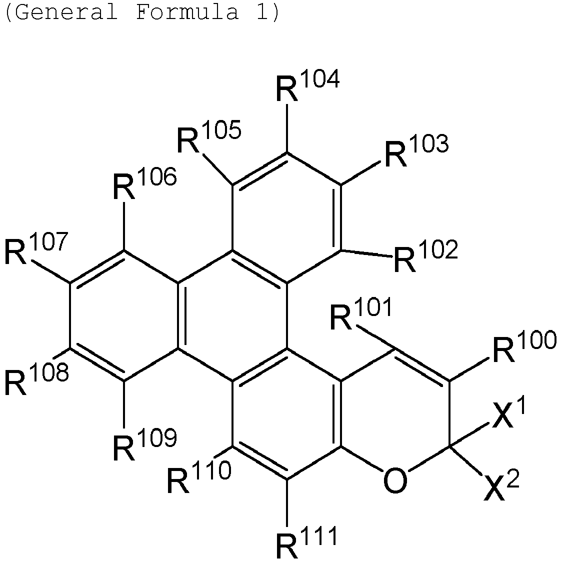

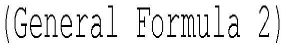

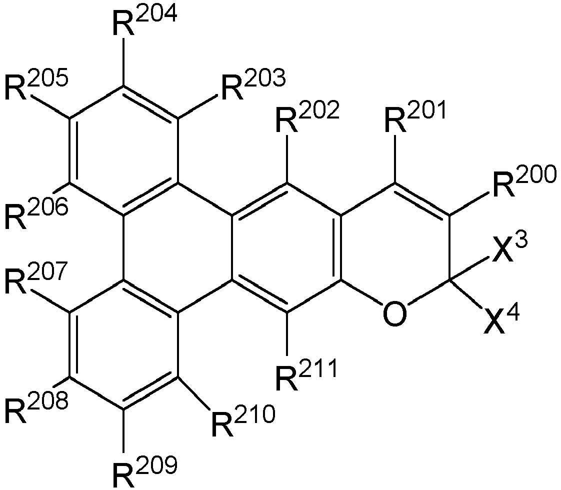

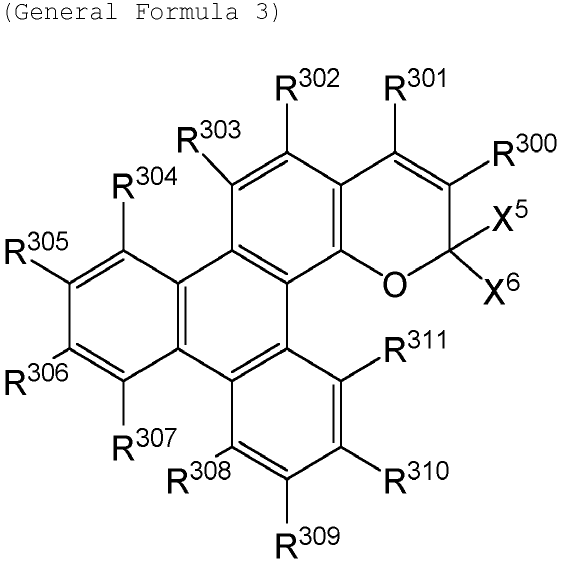

## Description

### [Technical Field]

The present invention relates to a photochromic compound, a photochromic composition, a photochromic article and eyeglasses.

### [Background Art]

A photochromic compound is a compound having a property of coloring under emission of light in a wavelength range having photoresponsivity and fading without light emission (photochromic properties). For example, PTL 1 and PTL 2 disclose naphthopyran compounds having photochromic properties.

### [Citation List]

### [Patent Literature]

[PTL 1] WO 2000/15631
[PTL 2] WO 1996/14596

### [Summary of Invention]

### [Technical Problem]

For example, photochromic properties can be imparted to optical articles such as spectacle lenses by a method of incorporating a photochromic compound into a substrate, a method of forming a layer containing a photochromic compound or the like.

As an example, a photochromic compound undergoes structural conversion into a colored component through an excited state when it receives light such as sunlight. The structure after structural conversion via light emission may be called a "colored component". On the other hand, the structure before light emission may be called a "colorless component". Here, regarding the colorless component, the term "colorless" is not limited to being completely colorless, and includes a case in which the color is lighter than that of the colored component. A photochromic compound in which absorption of visible light having a short wavelength around 380 nm is minimized in a colorless component state is preferable for providing an optical article that is less colored (for example, yellowish) in a usage environment such as indoors.

An object of one aspect of the present invention is to provide a photochromic compound in which absorption of visible light having a short wavelength is minimized in a colorless component state.

### [Solution to Problem]

One aspect of the present invention relates to a photochromic compound represented by the following General Formula 1.

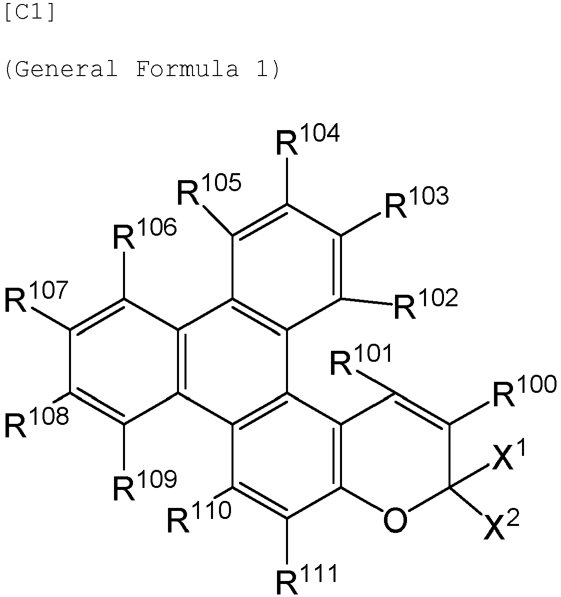

In General Formula 1, R¹⁰⁰ to R¹¹¹, and X¹ and X² each independently represent a hydrogen atom or a substituent, and two or more substituents may be bonded to form a ring structure.

Another aspect of the present invention relates to a photochromic compound represented by the following General Formula 2.

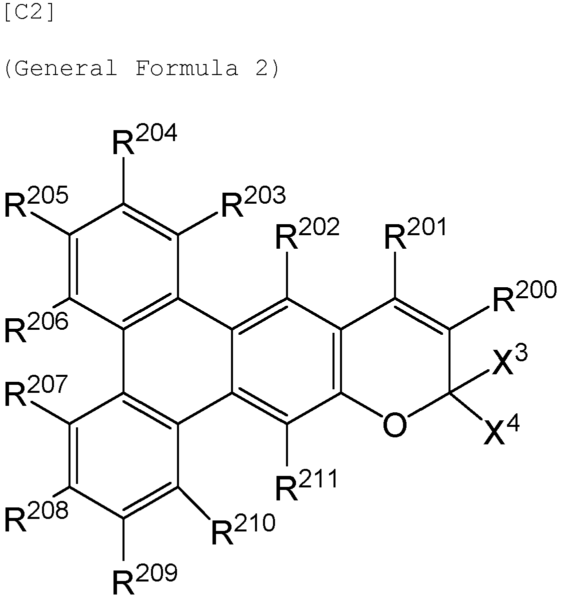

In General Formula 2, R²⁰⁰ to R²¹¹, and X³ and X⁴ each independently represent a hydrogen atom or a substituent, and two or more substituents may be bonded to form a ring structure.

Another aspect of the present invention relates to a photochromic compound represented by the following General Formula 3.

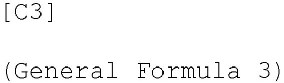

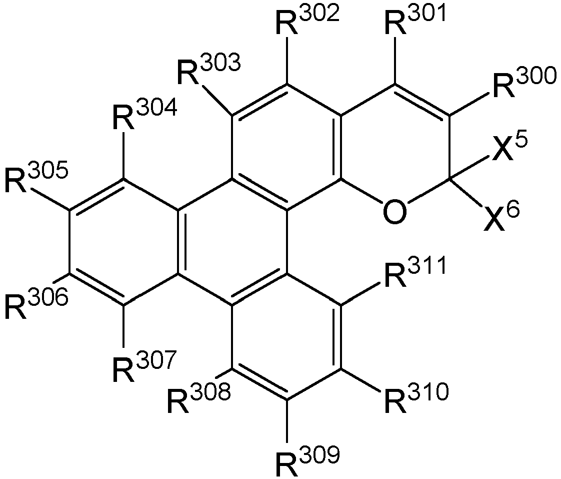

In General Formula 3, R³⁰⁰ to R³¹¹, and X⁵ and X⁶ each independently represent a hydrogen atom or a substituent, and two or more substituents may be bonded to form a ring structure.

The structures of General Formulae 1 to 3 are all colorless component structures.

In addition, one aspect of the present invention relates to a photochromic composition containing one or more photochromic compounds selected from the group consisting of photochromic compounds represented by General Formula 1, photochromic compounds represented by General Formula 2 and photochromic compounds represented by General Formula 3.

In addition, one aspect of the present invention relates to a photochromic article containing one or more photochromic compounds selected from the group consisting of photochromic compounds represented by General Formula 1, photochromic compounds represented by General Formula 2 and photochromic compounds represented by General Formula 3.

Photochromic compounds represented by General Formula 1, photochromic compounds represented by General Formula 2 and photochromic compounds represented by General Formula 3 are all pyran compounds having a triphenylene framework as a base framework. The inventors conducted extensive studies, and as a result, newly found that these photochromic compounds absorb less visible light having a short wavelength in a colorless component state.

### [Advantageous Effects of Invention]

According to one aspect of the present invention, it is possible to provide a photochromic compound in which absorption of visible light having a short wavelength is minimized in a colorless component state.

### [Brief Description of Drawings]

[Fig. 1]

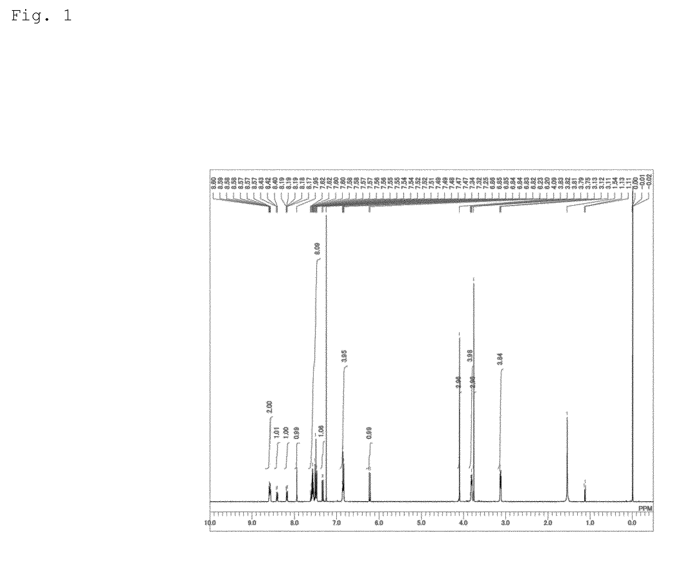

   Fig. 1 is an NMR chart of synthesized Exemplary Compound 1.
[Fig. 2]

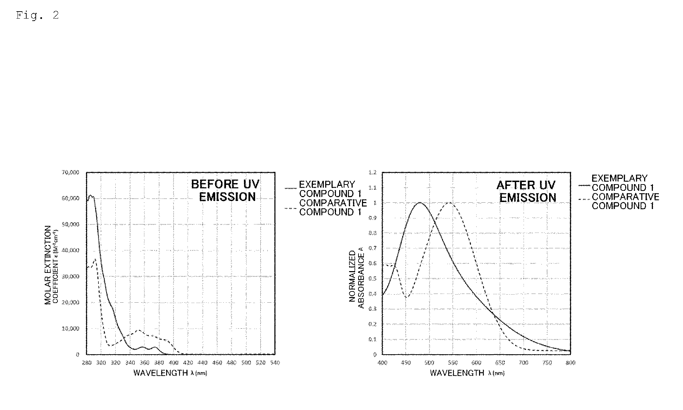

   Fig. 2 shows absorption spectrums of Exemplary Compound 1 and Comparative Compound 1 before and after UV emission.

### [Description of Embodiments]

In the present invention and this specification, the term "photochromic article" refers to an article containing a photochromic compound. The photochromic article according to one aspect of the present invention contains one or more photochromic compounds selected from the group consisting of photochromic compounds represented by General Formula 1, photochromic compounds represented by General Formula 2 and photochromic compounds represented by General Formula 3. The photochromic compound can be incorporated into a substrate of a photochromic article and/or can be incorporated into a photochromic layer in a photochromic article having a substrate and a photochromic layer. The term "photochromic layer" is a layer containing a photochromic compound.

In the present invention and this specification, the term "photochromic composition" refers to a composition containing a photochromic compound. The photochromic composition according to one aspect of the present invention contains one or more photochromic compounds selected from the group consisting of photochromic compounds represented by General Formula 1, photochromic compounds represented by General Formula 2 and photochromic compounds represented by General Formula 3, and can be used for producing a photochromic article according to one aspect of the present invention.

### [Photochromic Compound]

In General Formulae 1 to 3, R¹⁰⁰ to R¹¹¹, R²⁰⁰ to R²¹¹, R³⁰⁰ to R³¹¹ and X¹ to X⁶ each independently represent a hydrogen atom or a substituent. In respective general formulae, two or more substituents may be bonded to form a ring structure.

In the present invention and this specification, various substituents, that is, substituents that can be represented by any of R¹⁰⁰ to R¹¹¹, R²⁰⁰ to R²¹¹, R³⁰⁰ to R³¹¹ and X¹ to X⁶ in General Formulae 1 to 3, and additionally, substituents when respective groups to be described below have a substituent, may each independently represent
a substituent R^{m} selected from the group consisting of linear or branched alkyl groups having 1 to 18 carbon atoms such as a hydroxy group, methyl group, ethyl group, propyl group, butyl group, pentyl group, and hexyl group, monocyclic or multicyclic cycloaliphatic alkyl groups such as a bicyclic ring having 5 to 18 carbon atoms such as a cyclopentyl group and cyclohexyl group, linear or branched alkoxy groups having 1 to 24 constituent atoms such as a methoxy group, ethoxy group, and butoxy group, linear or branched perfluoroalkyl groups having 1 to 18 carbon atoms such as non-aromatic cyclic substituents having 1 to 24 constituent atoms and a trifluoromethyl group, linear or branched perfluoroalkoxy groups such as a trifluoromethoxy group, linear or branched alkylsulfide groups having 1 to 24 constituent atoms such as a methylsulfide group, ethylsulfide group, and butylsulfide group, aryl groups such as a phenyl group, naphthyl group, anthracenyl group, fluoranthenyl group, phenanthryl group, pyranyl group, perylenyl group, styryl group, and fluorenyl group, aryloxy groups such as a phenyloxy group, arylsulfide groups such as a phenylsulfide group, heteroaryl groups such as a pyridyl group, furanyl group, thienyl group, pyrrolyl group, benzofuranyl group, benzothiophenyl group, indolyl group, dibenzofuranyl group, dibenzothiophenyl group, carbazolyl group, diazolyl group, triazolyl group, quinolinyl group, phenothiazinyl group, phenoxazinyl group, phenazinyl group, thianthryl group, and acridinyl group, monoalkylamino groups such as an amino group (-NH₂) and monomethylamino group, dialkylamino groups such as a dimethylamino group, monoarylamino groups such as a monophenylamino group, diarylamino groups such as a diphenylamino group, cyclic amino groups such as a piperidino group, morpholino group, thiomorpholino group, tetrahydroquinolino group, and tetrahydroisoquinolino group, an ethynyl group, a mercapto group, a silyl group, a sulfonic acid group, an alkylsulfonyl group, a formyl group, a carboxy group, a cyano group and halogen atoms such as a fluorine atom, chlorine atom, bromine atom, and iodine atom; or
a substituent in which R^{m} is additionally substituted with one or more of the same or different R^{m}'s.

As an example of the substituent in which the above R^{m} is additionally substituted with one or more of the same or different R^{m}'s, a structure in which the terminal carbon atom of an alkoxy group is additionally substituted with an alkoxy group, and the terminal carbon atom of the alkoxy group is additionally substituted with an alkoxy group may be exemplified. In addition, as another example of the substituent in which the above R^{m} is additionally substituted with one or more of the same or different R^{m}'s, a structure in which two or more positions among five substitutable positions of a phenyl group are substituted with the same or different R^{m}' s may be exemplified. However, the present invention is not limited to such examples.

In the present invention and this specification, the terms "number of carbon atoms" and "number of constituent atoms" refer to the numbers including the number of carbon atoms or the number of atoms of the substituent with respect to a group having a substituent.

In addition, in the present invention and this specification, various substituents, that is, substituents that can be represented by any of R¹⁰⁰ to R¹¹¹, R²⁰⁰ to R²¹¹, R³⁰⁰ to R³¹¹ and X¹ to X⁶ in General Formulae 1 to 3, and additionally, substituents when respective groups to be described below have a substituent, may each independently represent a solubilizing group. In the present invention and this specification, the "solubilizing group" refers to a substituent that can contribute to increasing the compatibility with any liquid or a specific liquid. Examples of solubilizing groups include alkyl groups containing a linear, branched or cyclic structure having 4 to 50 carbon atoms, linear, branched or cyclic alkoxy groups having 4 to 50 constituent atoms, linear, branched or cyclic silyl groups having 4 to 50 constituent atoms, those in which some of the above groups are substituted with a silicon atom, sulfur atom, nitrogen atom, phosphorus atom or the like, and those obtained by combining two or more of the above groups, and a substituent that can contribute to promoting thermal motion of molecules of the compound according to inclusion of this substituent is preferable. A compound having a solubilizing group as a substituent can inhibit the distance between solute molecules from decreasing and prevent the solute from solidifying, and can lower the melting point and/or glass transition temperature of the solute and create a molecule aggregation state close to that of a liquid. Therefore, the solubilizing group can liquefy a solute or increase the solubility of the compound having this substituent in a liquid. In one aspect, the solubilizing group is preferably an n-butyl group, n-pentyl group, n-hexyl group, and n-octyl group which are a linear alkyl group, a tert-butyl group which is a branched alkyl group, or a cyclopentyl group and a cyclohexyl group which are a cyclic alkyl group.

The substituent is preferably a substituent selected from the group consisting of a methoxy group, ethoxy group, phenoxy group, methylsulfide group, ethylsulfide group, phenylsulfide group, trifluoromethyl group, phenyl group, naphthyl group, dibenzofuranyl group, dibenzothiophenyl group, carbazolyl group, phenothiazinyl group, phenoxazinyl group, phenazinyl group, acridinyl group, dimethylamino group, diphenylamino group, piperidino group, morpholino group, thiomorpholino group, cyano group and solubilizing group, and more preferably a substituent selected from the group consisting of a methoxy group, phenoxy group, methylsulfide group, phenylsulfide group, trifluoromethyl group, phenyl group, dimethylamino group, diphenylamino group, piperidino group, morpholino group, thiomorpholino group, cyano group and solubilizing group.

Specific examples of substituents that can be represented by R¹⁰⁰ to R¹¹¹ in General Formula 1, R²⁰⁰ to R²¹¹ in General Formula 2, and R³⁰⁰ to R³¹¹ in General Formula 3 include substituents contained in exemplary compounds listed below.

X¹ and X² in General Formula 1, X³ and X⁴ in General Formula 2 and X⁵ and X⁶ in General Formula 3 each independently represent a hydrogen atom or a substituent, and preferably represent a substituent. Preferably, such substituents represent a substituted or unsubstituted phenyl group, substituted or unsubstituted naphthyl group, substituted or unsubstituted fluorenyl group, substituted or unsubstituted benzofluorenyl group, substituted or unsubstituted fluoranthenyl group, substituted or unsubstituted dibenzofuranyl group or substituted or unsubstituted dibenzothiophenyl group. More preferably, B7 and B8 each independently represent an unsubstituted phenyl group or substituted phenyl group. The substituted phenyl group can contain, for example, one or more substituents selected from the group consisting of a methoxy group, methylsulfide group, amino group, dimethylamino group, piperidino group, morpholino group, thiomorpholino group, phenyl group, fluorine atom, chlorine atom, bromine atom, iodine atom, trifluoromethyl group, cyano group and substituents contained in exemplary compounds listed below. In addition, when the phenyl group has a plurality of substituents, two or more of these substituents may be bonded to form a ring. Specific examples of rings to be formed include rings included in exemplary compounds listed below. In one aspect, the number of substituents of the substituted phenyl group is preferably 1 or 2, and more preferably 1. The substitution position of the substituent in the substituted phenyl group may be a position that is an ortho position, meta position or para position with respect to a carbon atom to which X¹ and X² in General Formula 1 are bonded, a carbon atom to which X³ and X⁴ in General Formula 2 are bonded, and a carbon atom to which X⁵ and X⁶ in General Formula 3 are bonded, and is preferably a para position and/or meta position, and more preferably a para position.

Photochromic compounds represented by General Formula 1, photochromic compounds represented by General Formula 2 and photochromic compounds represented by General Formula 3 all can be used for producing photochromic articles. Specific examples of these compounds include the following compounds. However, the present invention is not limited to the following exemplary compounds.

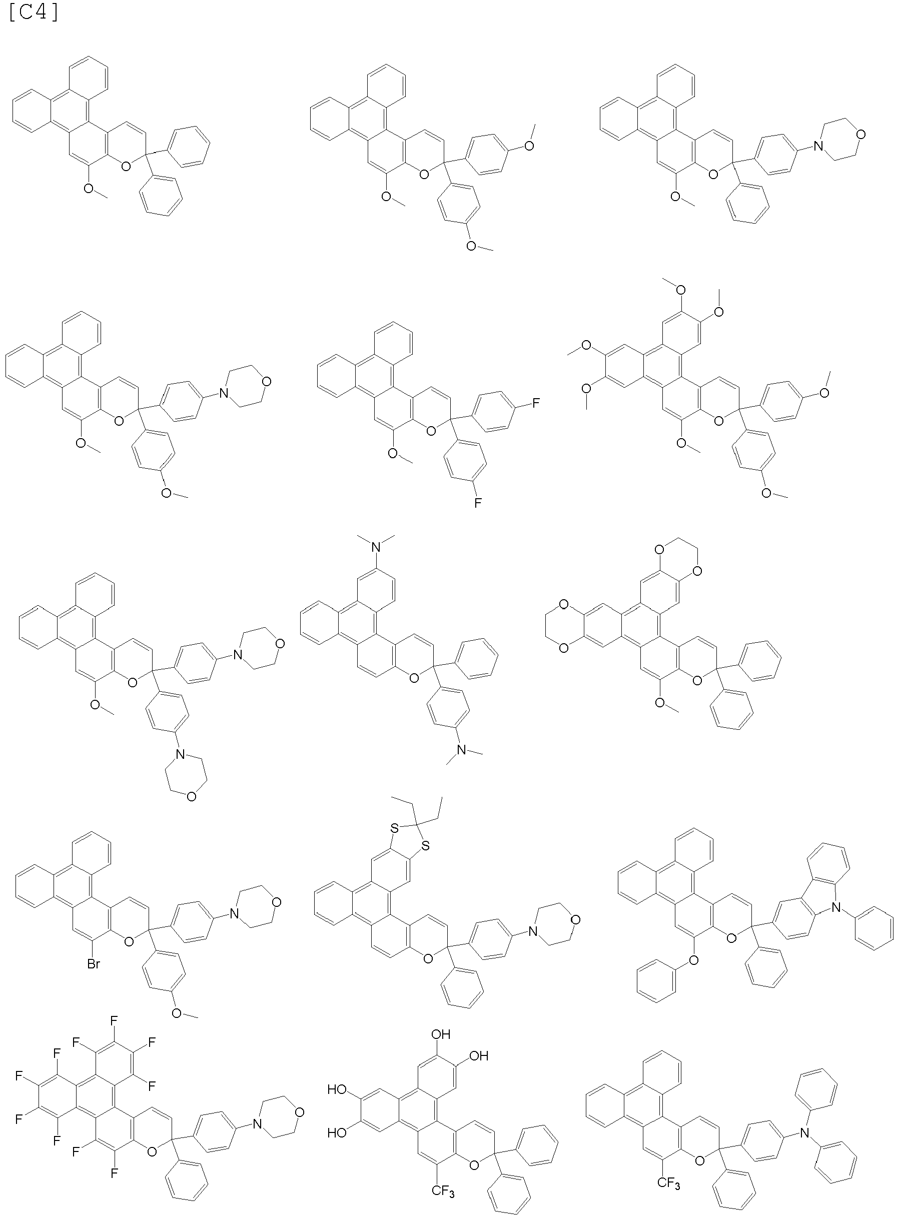

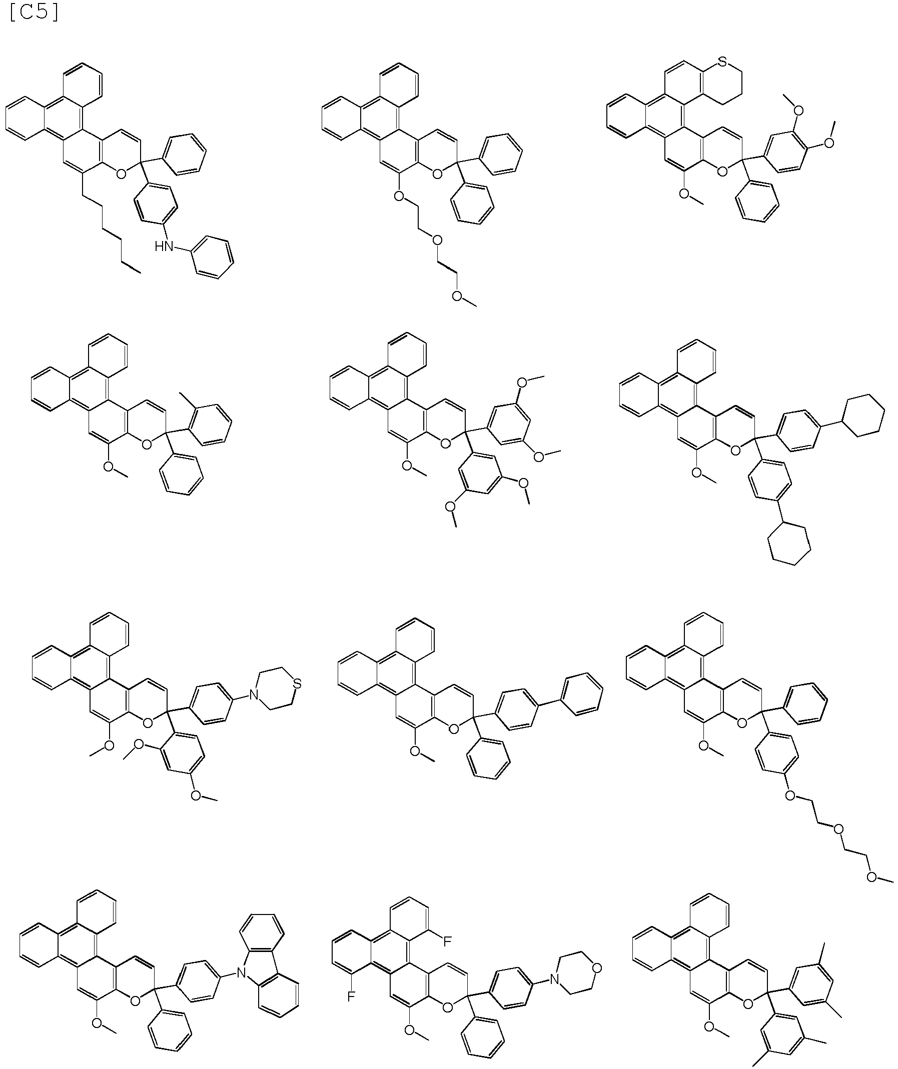

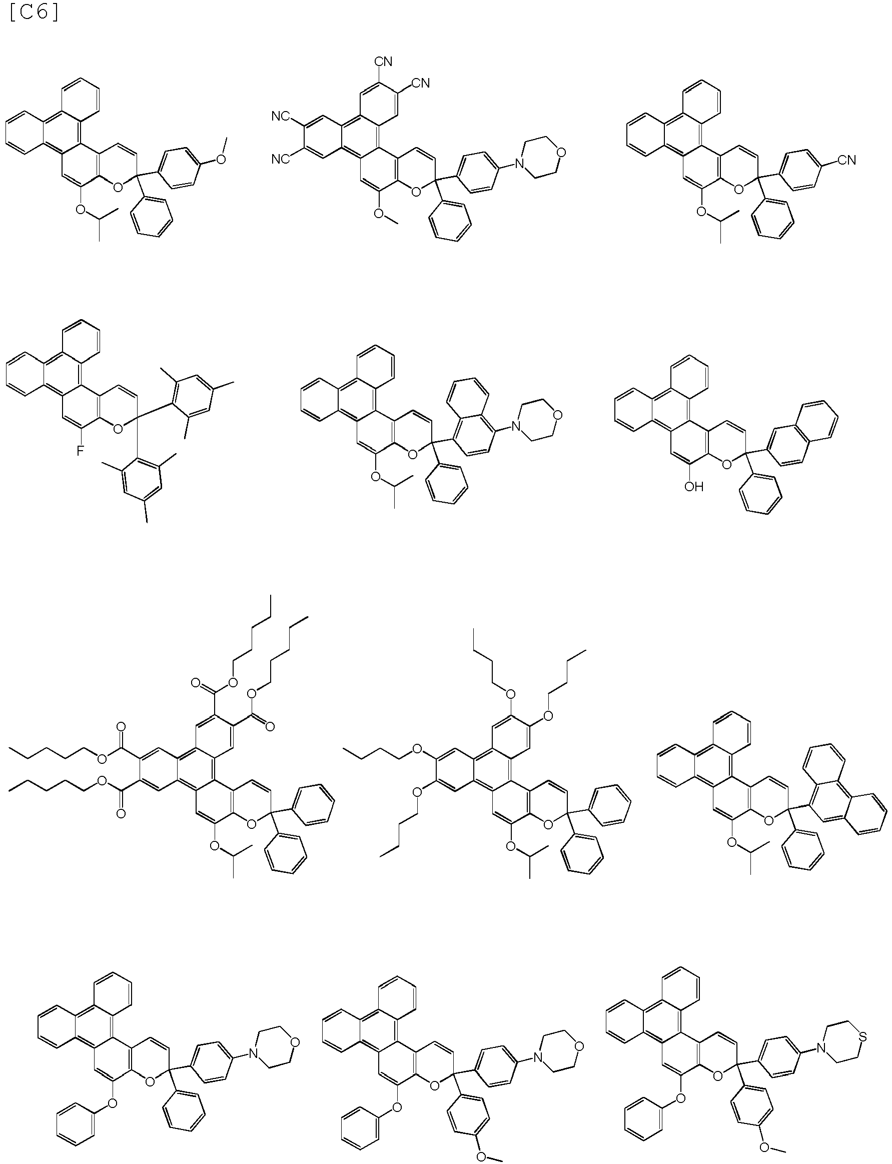

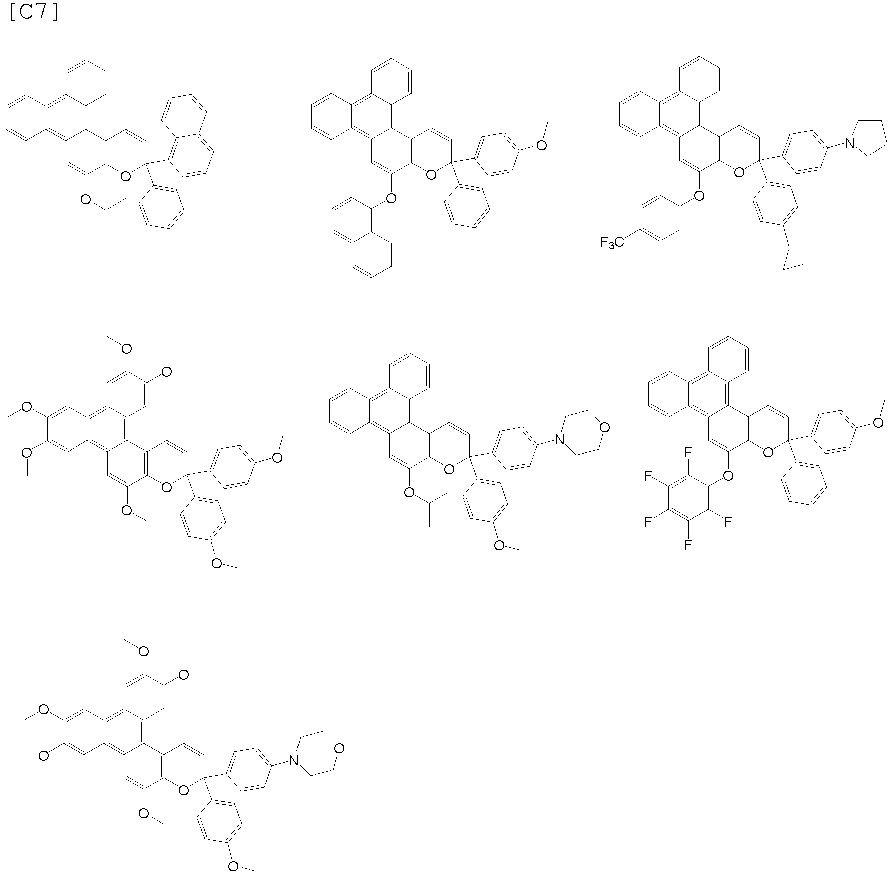

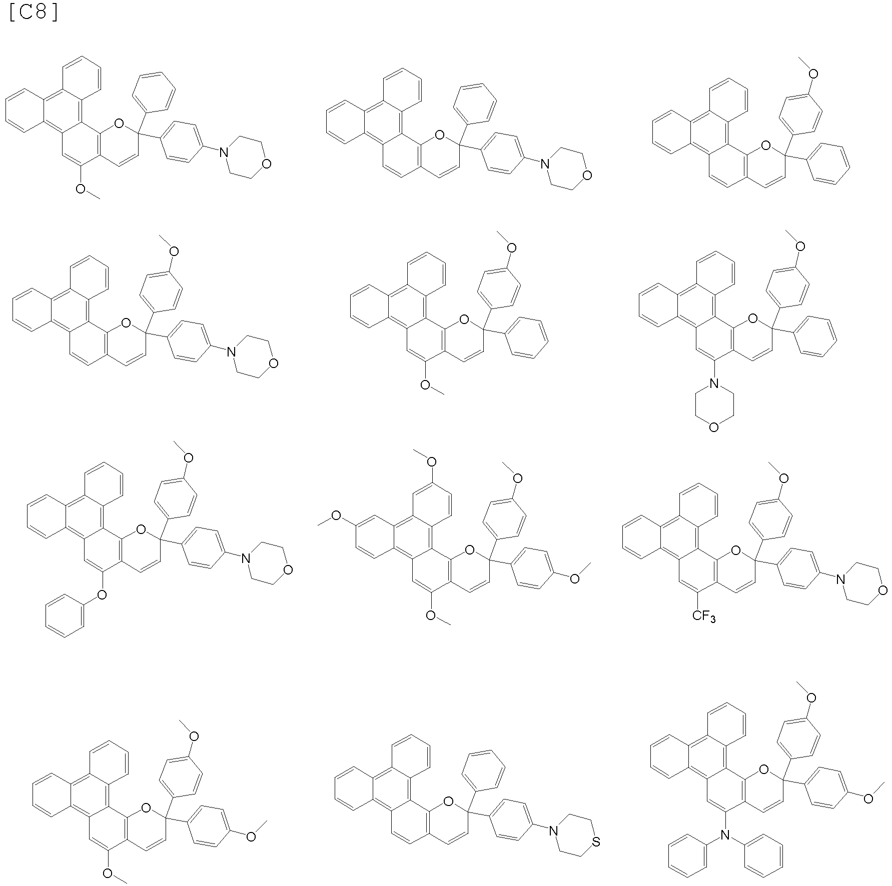

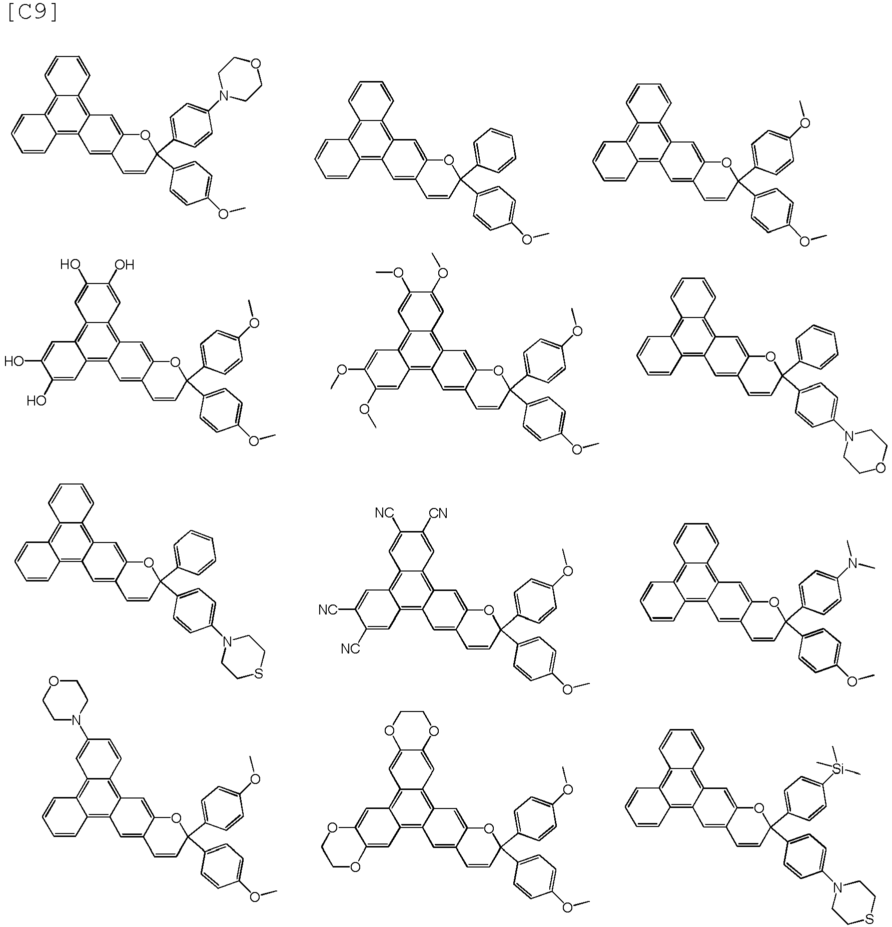

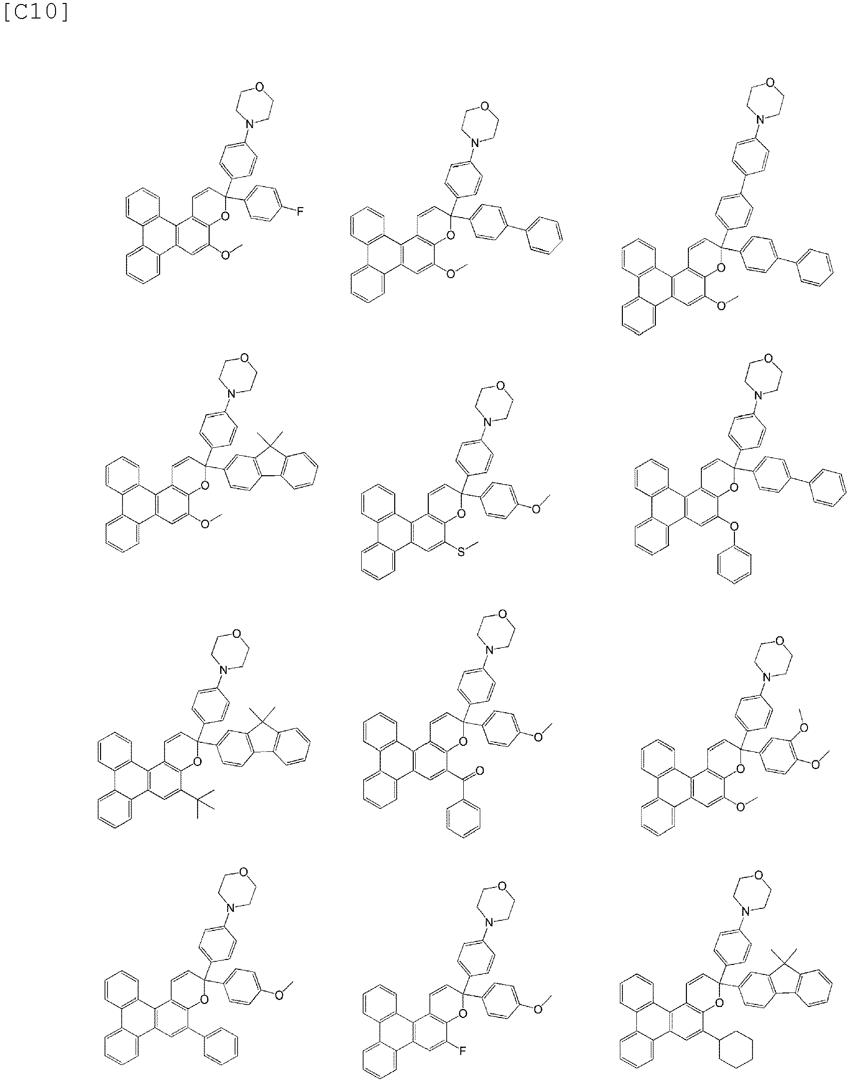

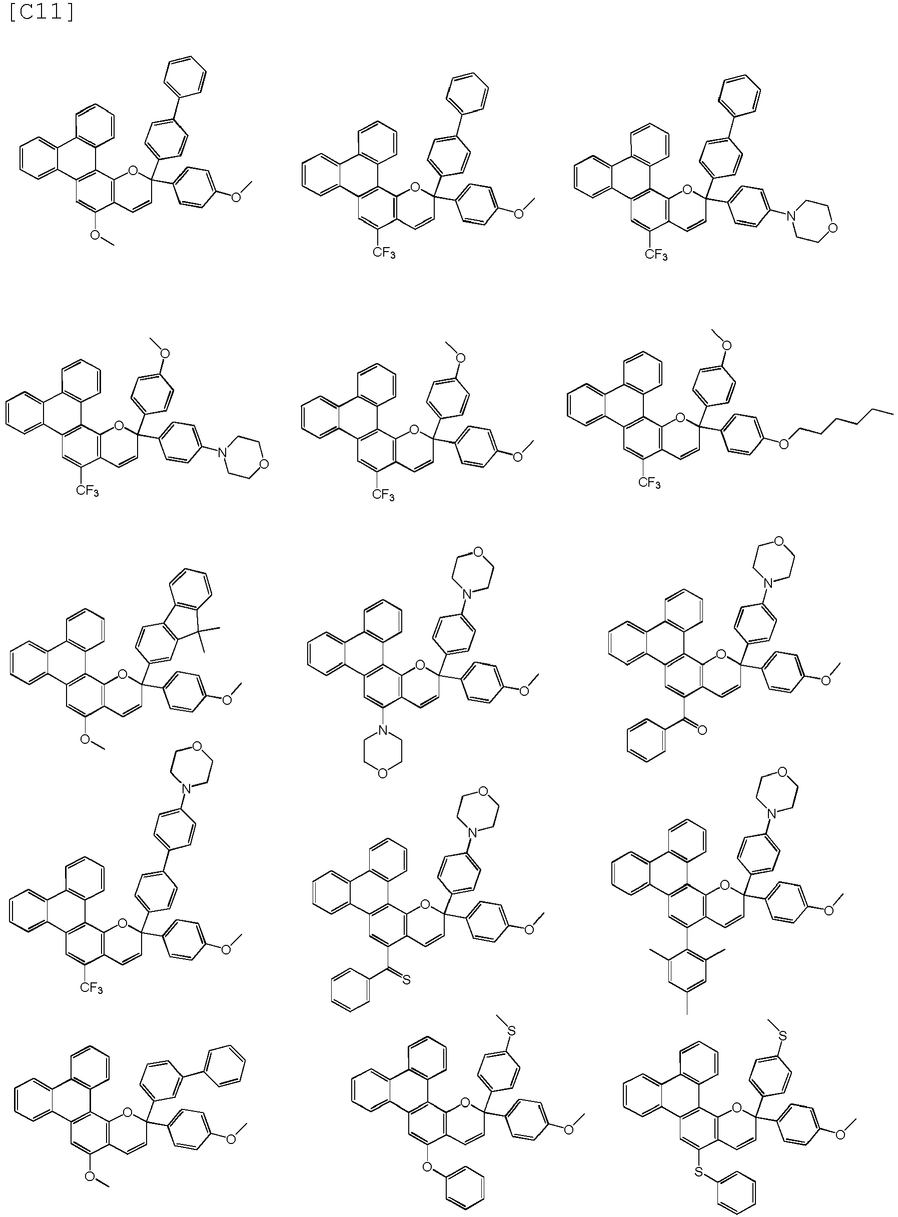

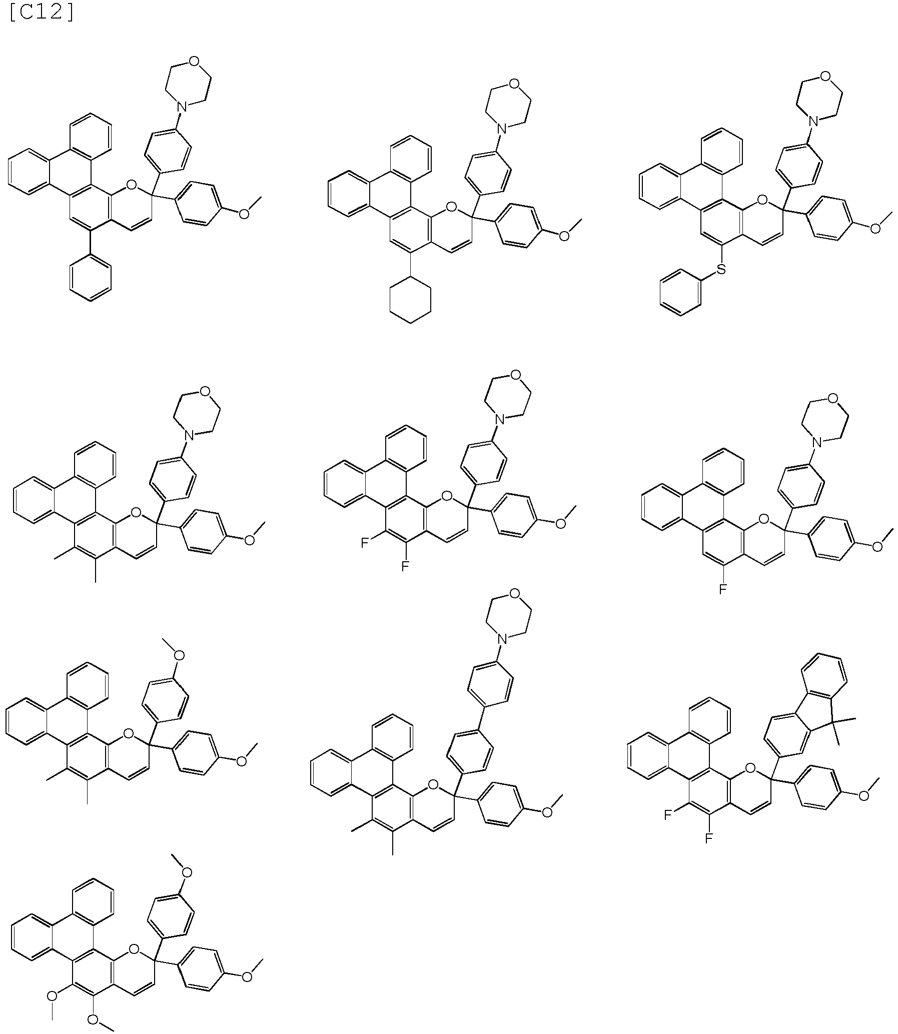

The photochromic compounds represented by General Formula 1 can be synthesized by a known method. For the synthesis method, for example, the following documents can be referred to. Japanese Patent No. 4884578, US 2006/0226402A1, US 2006/0228557A1, US 2008/0103301A1, US 2011/0108781A1, US 2011/0108781A1, U.S. Patent No. 7527754, U.S. Patent No. 7556751, WO 2001/60811A1, WO 2013/086248A1, WO 1996/014596A1, WO 2001/019813A1, WO 1995/16215A1, U.S. Patent No. 5656206 and WO 2011/016582A1.

### [Photochromic Composition and Photochromic Article]

One aspect of the present invention relates to a photochromic composition containing one or more photochromic compounds selected from the group consisting of photochromic compounds represented by General Formula 1, photochromic compounds represented by General Formula 2 and photochromic compounds represented by General Formula 3.

In addition, one aspect of the present invention relates to a photochromic article containing one or more photochromic compounds selected from the group consisting of photochromic compounds represented by General Formula 1, photochromic compounds represented by General Formula 2 and photochromic compounds represented by General Formula 3.

The photochromic composition and the photochromic article can contain only one photochromic compound selected from the group consisting of photochromic compounds represented by General Formula 1, photochromic compounds represented by General Formula 2 and photochromic compounds represented by General Formula 3 or two or more (for example, two or more and four or less) thereof. The photochromic article and the photochromic composition can contain, for example, about 0.1 to 15.0 mass% of a photochromic compound selected from the group consisting of photochromic compounds represented by General Formula 1, photochromic compounds represented by General Formula 2 and photochromic compounds represented by General Formula 3 (a total content thereof when it contains a plurality of compounds) with respect to a total amount of 100 mass% thereof. However, the present invention is not limited to the above range.

The photochromic article can have at least a substrate. In one aspect, the photochromic compound can be included in the substrate of the photochromic article. The photochromic article can have a substrate and a photochromic layer, and the substrate and/or the photochromic layer can contain one or more photochromic compounds represented by General Formula 1. In the substrate and the photochromic layer, in one aspect, the photochromic compound can be contained only in the substrate, in another aspect, the photochromic compound can be contained only in the photochromic layer, and in still another aspect, the photochromic compound can be contained in the substrate and the photochromic layer. In addition, the substrate and the photochromic layer can contain, as a photochromic compound, only the above photochromic compound or one or more other photochromic compounds. Examples of other photochromic compounds include azobenzenes, spiropyrans, spirooxazines, naphthopyrans, indenonaphthopyrans, phenanthropyrans, hexaallylbisimidazoles, donor-acceptor Stenhouse adducts (DASA), salicylidene anilines, dihydropyrenes, anthracene dimers, fulgides, diarylethenes, phenoxynaphthacenequinones, and stilbenes.

### <Substrate>

The photochromic article can contain a substrate selected according to the type of the photochromic article. Examples of substrates include spectacle lens substrates such as a plastic lens substrate and a glass lens substrate. The glass lens substrate can be, for example, a lens substrate made of inorganic glass. Examples of plastic lens substrates include styrene resins such as (meth)acrylic resins, allyl carbonate resins such as a polycarbonate resin, allyl resin, and diethylene glycol bisallyl carbonate resin (CR-39), vinyl resins, polyester resins, polyether resins, urethane resins obtained by reacting an isocyanate compound and a hydroxy compound such as diethylene glycol, thiourethane resins obtained by reacting an isocyanate compound and a polythiol compound, and a cured product obtained by curing a curable composition containing a (thio)epoxy compound having one or more disulfide bonds in the molecule (generally referred to as a transparent resin). As the lens substrate, an undyed substrate (colorless lens) may be used or a dyed substrate (dyed lens) may be used. The refractive index of the lens substrate may be, for example, about 1.50 to 1.75. However, the refractive index of the lens substrate is not limited to the above range, and may be within the above range or may be above or below outside the above range. Here, the refractive index refers to a refractive index for light having a wavelength of 500 nm. In addition, the lens substrate may be a lens having refractive power (so-called prescription lens) or a lens having no refractive power (so-called non-prescription lens) .

For example, the photochromic composition can be a polymerizable composition.

In the present invention and this specification, the "polymerizable composition" is a composition containing one or more polymerizable compounds. A polymerizable composition containing at least one or more photochromic compounds selected from the group consisting of photochromic compounds represented by General Formula 1, photochromic compounds represented by General Formula 2 and photochromic compounds represented by General Formula 3, and one or more polymerizable compounds can be molded by a known molding method to produce a cured product of such a polymerizable composition.

Such a cured product can be included as a substrate in the photochromic article and/or can be included as a photochromic layer.

The curing treatment can be light emission and/or a heat treatment. The polymerizable compound is a compound having a polymerizable group, and as the polymerization reaction of the polymerizable compound proceeds, the polymerizable composition can be cured to form a cured product. The polymerizable composition can further contain one or more additives (for example, a polymerization initiator).

The spectacle lens may include various lenses such as a single focus lens, a multifocal lens, and a progressive power lens. The type of the lens is determined by the surface shape of both sides of the lens substrate. In addition, the surface of the lens substrate may be a convex surface, a concave surface, or a flat surface. In a general lens substrate and spectacle lens, the object-side surface is a convex surface and the eyeball-side surface is a concave surface. However, the present invention is not limited thereto. The photochromic layer may be generally provided on the object-side surface of the lens substrate, or may be provided on the eyeball-side surface.

### <Photochromic Layer>

The photochromic layer can be a layer that is directly provided on the surface of the substrate or indirectly provided via one or more other layers. The photochromic layer can be, for example, a cured layer obtained by curing a polymerizable composition. A photochromic layer can be formed as a cured layer obtained by curing a polymerizable composition containing at least one or more photochromic compounds selected from the group consisting of photochromic compounds represented by General Formula 1, photochromic compounds represented by General Formula 2 and photochromic compounds represented by General Formula 3, and one or more polymerizable compounds. For example, when such a polymerizable composition is directly applied to the surface of the substrate or applied to the surface of the layer provided on the substrate, and a curing treatment is performed on the applied polymerizable composition, a photochromic layer can be formed as a cured layer containing one or more photochromic compounds selected from the group consisting of photochromic compounds represented by General Formula 1, photochromic compounds represented by General Formula 2 and photochromic compounds represented by General Formula 3. As the coating method, known coating methods such as a spin coating method, a dip coating method, a spray coating method, an inkjet method, a nozzle coating method, and a slit coating method can be used. The curing treatment can be light emission and/or a heat treatment. The polymerizable composition can further contain one or more additives (for example, a polymerization initiator) in addition to one or more polymerizable compounds. As the polymerization reaction of the polymerizable compound proceeds, the polymerizable composition can be cured to form a cured layer.

The thickness of the photochromic layer may be, for example, 5 um or more, 10 um or more or 20 um or more, and may be, for example, 80 um or less, 70 um or less or 50 um or less.

### <Polymerizable Compound>

In the present invention and this specification, the term polymerizable compound refers to a compound having one or more polymerizable groups in one molecule, and the term "polymerizable group" refers to a reactive group that can undergo a polymerization reaction. Examples of polymerizable groups include an acryloyl group, methacryloyl group, vinyl group, vinyl ether group, epoxy group, thiol group, oxetane group, hydroxy group, carboxy group, amino group, and isocyanate group.

Examples of polymerizable compounds that can be used to form the above substrate and the above photochromic layer include the following compounds.

### (Episulfide Compound)

The episulfide compound is a compound having two or more episulfide groups in one molecule. The episulfide group is a polymerizable group that can undergo ring-opening polymerization. Specific examples of episulfide compounds include bis(1,2-epithioethyl)sulfide, bis(1,2-epithioethyl)disulfide, bis(2,3-epithiopropyl)sulfide, bis(2,3-epithiopropylthio)methane, bis(2,3-epithiopropyl)disulfide, bis(2,3-epithiopropyldithio)methane, bis(2,3-epithiopropyldithio)ethane, bis(6,7-epithio-3,4-dithiaheptyl)sulfide, bis(6,7-epithio-3,4-dithiaheptyl)disulfide, 1,4-dithiane-2,5-bis(2,3-epithiopropyldithiomethyl), 1,3-bis(2,3-epithiopropyldithiomethyl)benzene, 1,6-bis(2,3-epithiopropyldithiomethyl)-2-(2,3-epithiopropyldithioethylthio)-4-thiahexane, 1,2,3-tris(2,3-epithiopropyldithio)propane, 1,1,1,1-tetrakis(2,3-epithiopropyldithiomethyl)methane, 1,3-bis(2,3-epithiopropyldithio)-2-thiapropane, 1,4-bis(2,3-epithiopropyldithio)-2,3-dithiabutane, 1,1,1-tris(2,3-epithiopropyldithio)methane, 1,1,1-tris(2,3-epithiopropyldithiomethylthio)methane, 1,1,2,2-tetrakis(2,3-epithiopropyldithio)ethane, 1,1,2,2-tetrakis(2,3-epithiopropyldithiomethylthio)ethane, 1,1,3,3-tetrakis(2,3-epithiopropyldithio)propane, 1,1,3,3-tetrakis(2,3-epithiopropyldithiomethylthio)propane, 2-[1,1-bis(2,3-epithiopropyldithio)methyl]-1,3-dithietane, and 2-[1,1-bis(2,3-epithiopropyldithiomethylthio)methyl]-1,3-dithietane.

### (Thietanyl Compound)

The thietanyl compound is a thietane compound having two or more thietanyl groups in one molecule. The thietanyl group is a polymerizable group that can undergo ring-opening polymerization. Some thietanyl compounds have an episulfide group together with a plurality of thietanyl groups. Such compounds are listed as examples in the above episulfide compound. Other thietanyl compounds include metal-containing thietane compounds having metal atoms in the molecule and non-metallic thietane compounds which contain no metal.

Specific examples of non-metallic thietane compounds include bis(3-thietanyl)disulfide, bis(3-thietanyl)sulfide, bis(3-thietanyl)trisulfide, bis(3-thietanyl)tetrasulfide, 1,4-bis(3-thietanyl)-1,3,4-trithibutane, 1,5-bis(3-thietanyl)-1,2,4,5-tetrathiapentane, 1,6-bis(3- thietanyl)-1,3,4,6-tetrathiahexane, 1,6-bis(3-thietanyl)-1,3,5,6-tetrathiahexane, 1,7-bis(3-thietanyl)-1,2,4,5,7-pentathiaheptane, 1,7-bis(3-thietanylthio)-1,2,4,6,7-pentathiaheptane, 1,1-bis(3-thietanylthio)methane, 1,2-bis(3-thietanylthio)ethane, 1,2,3-tris(3-thietanylthio)propane, 1,8-bis(3-thietanylthio)-4-(3-thietanylthiomethyl)-3,6-dithiaoctane, 1,11-bis(3-thietanylthio)-4,8-bis(3-thietanylthiomethyl)-3,6,9-trithiundecane, 1,11-bis(3-thietanylthio)-4,7-bis(3-thietanylthiomethyl)-3,6,9-trithiundecane, 1,11-bis(3-thietanylthio)-5,7-bis(3-thietanylthiomethyl)-3,6,9-trithiundecane, 2,5-bis(3-thietanylthiomethyl)-1,4-dithiane, 2,5-bis[[2-(3-thietanylthio)ethyl]thiomethyl]-1,4-dithiane, 2,5-bis(3-thietanylthiomethyl)-2,5-dimethyl-1,4-dithiane, bis-thietanylsulfide, bis(thietanylthio)methane, 3-[<(thietanylthio)methylthio>methylthio]thietane, bis-thietanyl disulfide, bis-thietanyl trisulfide, bis-thietanyl tetrasulfide, bis-thietanyl pentasulfide, 1,4-bis(3-thietanyldithio)-2,3-dithibutane, 1,1,1-tris(3-thietanyldithio)methane, 1,1,1-tris(3-thietanyldithiomethylthio)methane, 1,1,2,2-tetrakis(3-thietanyldithio)ethane, and 1,1,2,2-tetrakis(3-thietanyldithiomethylthio)ethane.

Examples of metal-containing thietane compounds include those containing Group 14 atoms such as Sn atoms, Si atoms, Ge atoms, and Pb atoms, Group 4 elements such as Zr atoms and Ti atoms, Group 13 atoms such as Al atoms, and Group 12 atoms such as Zn atoms, as metal atoms in the molecule. Specific examples thereof include alkylthio(thietanylthio)tin, bis(alkylthio)bis (thietanylthio)tin, alkylthio(alkylthio)bis(thietanylthio)tin, bis(thietanylthio)cyclic dithiotin compounds, and alkyl(thietanylthio)tin compounds.

Specific examples of alkylthio(thietanylthio)tin include methylthiotris(thietanylthio)tin, ethylthiotris(thietanylthio)tin, propylthiotris(thietanylthio)tin, and isopropylthiotris(thietanylthio)tin.

Specific examples of bis(alkylthio)bis(thietanylthio)tin include bis(methylthio)bis(thietanylthio)tin, bis(ethylthio)bis (thietanylthio)tin, bis(propylthio)bis(thietanylthio)tin, and bis(isopropylthio)bis (thietanylthio)tin.

Specific examples of alkylthio(alkylthio)bis(thietanylthio)tin include ethylthio(methylthio)bis(thietanylthio)tin, methylthio(propylthio)bis(thietanylthio)tin, isopropylthio(methylthio)bis(thietanylthio)tin, ethylthio(propylthio)bis(thietanylthio)tin, ethylthio(isopropylthio)bis(thietanylthio)tin, and isopropylthio(propylthio)bis(thietanylthio)tin.

Specific examples of bis(thietanylthio)cyclic dithiotin compounds include bis(thietanylthio)dithiastannetane, bis(thietanylthio)dithiastannolane, bis(thietanylthio)dithiastannolane, and bis(thietanylthio)trithiastannocane.

Specific examples of alkyl(thietanylthio)tin compounds include methyltris(thietanylthio)tin, dimethylbis(thietanylthio)tin, butyltris(thietanylthio)tin, tetrakis(thietanylthio)tin, tetrakis(thietanylthio)germanium, and tris(thietanylthio)bismuth.

### (Polyamine compound)

The polyamine compound is a compound having two or more NH₂ groups in one molecule, and can form a urea bond according to a reaction with a polyisocyanate and can form a thiourea bond according to a reaction with a polyisothiocyanate. Specific examples of polyamine compounds include ethylenediamine, hexamethylenediamine, isophoronediamine, nonamethylenediamine, undecamethylenediamine, dodecamethylenediamine, metaxylenediamine, 1,3-propanediamine, putrescine, 2-(2-aminoethylamino)ethanol, diethylenetriamine, p-phenylenediamine, m-phenylenediamine, melamine, and 1,3,5-benzenetriamine.

### (Epoxy Compound)

The epoxy compound is a compound having an epoxy group in the molecule. The epoxy group is a polymerizable group that can undergo ring-opening polymerization. The epoxy compounds are generally classified into aliphatic epoxy compounds, alicyclic epoxy compounds and aromatic epoxy compounds.

Specific examples of aliphatic epoxy compounds include ethylene oxide, 2-ethyloxirane, butyl glycidyl ether, phenyl glycidyl ether, 2,2'-methylenebisoxirane, 1,6-hexanediol diglycidyl ether, ethylene glycol diglycidyl ether, diethylene glycol diglycidyl ether, triethylene glycol diglycidyl ether, tetraethylene glycol diglycidyl ether, nonaethylene glycol diglycidyl ether, propylene glycol diglycidyl ether, dipropylene glycol diglycidyl ether, tripropylene glycol diglycidyl ether, tetrapropylene glycol diglycidyl ether, nonapropylene glycol diglycidyl ether, neopentyl glycol diglycidyl ether, trimethylolpropane triglycidyl ether, glycerol triglycidyl ether, diglycerol tetraglycidyl ether, pentaerythritol tetraglycidyl ether, and tris(2-hydroxyethyl)isocyanurate triglycidyl ether.

Specific examples of alicyclic epoxy compounds include isophoronediol diglycidyl ether and bis-2,2-hydroxycyclohexylpropane diglycidyl ether.

Specific examples of aromatic epoxy compounds include resole syndiglycidyl ether, bisphenol A diglycidyl ether, bisphenol F diglycidyl ether, bisphenol S diglycidyl ether, diglycidyl orthophthalate, phenol novolac polyglycidyl ether, and cresol novolac polyglycidyl ether.

In addition, in addition to the above examples, an epoxy compound having a sulfur atom in the molecule together with an epoxy group can be used. Such epoxy compounds containing sulfur atoms include linear aliphatic compounds and cycloaliphatic compounds.

Specific examples of linear aliphatic epoxy compounds containing sulfur atoms include bis(2,3-epoxypropyl)sulfide, bis(2,3-epoxypropyl)disulfide, bis(2,3-epoxypropylthio)methane, 1,2-bis(2,3-epoxypropylthio)ethane, 1,2-bis(2,3-epoxypropylthio)propane, 1,3-bis(2,3-epoxypropylthio)propane, 1,3-bis(2,3-epoxypropylthio)-2-methylpropane, 1,4-bis(2,3-epoxypropylthio)butane, 1,4-bis(2,3-epoxypropylthio)-2-methylbutane, 1,3-bis(2,3-epoxypropylthio)butane, 1,5-bis(2,3-epoxypropylthio)pentane, 1,5-bis(2,3-epoxypropylthio)-2-methylpentane, 1,5-bis(2,3-epoxypropylthio)-3-thiapentane, 1,6-bis(2,3-epoxypropylthio)hexane, 1,6-bis(2,3-epoxypropylthio)-2-methylhexane, 3,8-bis(2,3-epoxypropylthio)-3,6-dithia octane, 1,2,3-tris(2,3-epoxypropylthio)propane, 2,2-bis(2,3-epoxypropylthio)-1,3-bis(2,3-epoxypropylthiomethyl)propane, and 2,2-bis(2,3-epoxypropylthiomethyl)-1-(2,3-epoxypropylthio)butane.

Specific examples of cycloaliphatic epoxy compounds containing sulfur atoms include 1,3-bis(2,3-epoxypropylthio)cyclohexane, 1,4-bis(2,3-epoxypropylthio)cyclohexane, 1,3-bis(2,3-epoxypropylthiomethyl)cyclohexane, 1,4-bis(2,3-epoxypropylthiomethyl)cyclohexane, 2,5-bis(2,3-epoxypropylthiomethyl)-1,4-dithiane, 2,5-bis[<2-(2,3-epoxypropylthio)ethyl>thiomethyl]-1,4-dithiane, and 2,5-bis(2,3-epoxypropylthiomethyl)-2,5-dimethyl-1,4-dithiane.

### (Compound having radically polymerizable group)

The compound having a radically polymerizable group has a polymerizable group that can undergo radical polymerization. Examples of radically polymerizable groups include an acryloyl group, methacryloyl group, allyl group, and vinyl group.

In the following, a compound having a polymerizable group selected from the group consisting of acryloyl groups and methacryloyl groups will be referred to as a "(meth)acrylate compound". Specific examples of (meth)acrylate compounds include ethylene glycol di(meth)acrylate, diethylene glycol di(meth)acrylate, triethylene glycol di(meth)acrylate, tetraethylene glycol di(meth)acrylate, polyethylene glycol di(meth)acrylate, propylene glycol di(meth)acrylate, dipropylene glycol di(meth)acrylate, tripropylene glycol di(meth)acrylate, polypropylene glycol di(meth)acrylate, neopentyl glycol di(meth)acrylate, ethylene glycol bisglycidyl(meth)acrylate), bisphenol A di(meth)acrylate, 2,2-bis(4-(meth)acryloxyethoxyphenyl)propane, 2,2-bis(4-(meth)acryloxydiethoxyphenyl)propane, 2,2-bis(4-(meth)acryloyloxyethoxyphenyl)propane, 2,2-bis(3,5-dibromo-4-(meth)acryloyloxyethoxyphenyl)propane, 2,2-bis(4-(meth)acryloyloxydipropoxyphenyl)propane, bisphenol F di(meth)acrylate, 1,1-bis(4-(meth)acryloxyethoxyphenyl)methane, 1,1-bis(4-(meth)acryloxydiethoxyphenyl)methane, dimethyloltricyclodecane di(meth)acrylate, trimethylolpropane tri(meth)acrylate, ditrimethylolpropane tetra(meth)acrylate, glycerol di(meth)acrylate, pentaerythritol tri(meth)acrylate, pentaerythritol tetra(meth)acrylate, methylthio(meth)acrylate, phenylthio(meth)acrylate, benzylthio(meth)acrylate, xylylene dithiol di(meth)acrylate, mercaptoethylsulfide di(meth)acrylate, and difunctional urethane (meth)acrylate.

Specific examples of compounds having an allyl group (allyl compound) include allyl glycidyl ether, diallyl phthalate, diallyl terephthalate, diallyl isophthalate, diallyl carbonate, diethylene glycol bisallyl carbonate, methoxy polyethylene glycol allyl ether, polyethylene glycol allyl ether, methoxy polyethylene glycol-polypropylene glycol allyl ether, butoxy polyethylene glycol-polypropylene glycol allyl ether, methacryloyloxy polyethylene glycol-polypropylene glycol allyl ether, phenoxy polyethylene glycol allyl ether, and methacryloyloxy polyethylene glycol allyl ether.

Examples of compounds having a vinyl group (vinyl compound) include α-methylstyrene, α-methylstyrene dimer, styrene, chlorostyrene, methylstyrene, bromostyrene, dibromostyrene, divinylbenzene, and 3,9-divinylspirobi (m-dioxane).

The photochromic article can include one or more layers known as functional layers of the photochromic article such as a protective layer for improving the durability of the photochromic article, an antireflection layer, a water-repellent or hydrophilic antifouling layer, a defogging layer, and a primer layer for improving adhesion between layers at any position.

The photochromic article can be an optical article. One form of the optical article is a spectacle lens. Such a spectacle lens can also be called a photochromic lens or a photochromic spectacle lens.

In addition, as one form of the optical article, a goggle lens, a visor (cap) part of a sun visor, a shield member of a helmet and the like may be exemplified. The photochromic composition which is a polymerizable composition is applied to the substrate for these optical articles, a curing treatment is performed on the applied composition, a photochromic layer is formed, and thereby an optical article having an anti-glare function can be obtained.

### [Eyeglasses]

One aspect of the present invention relates to eyeglasses having a spectacle lens that is one form of the photochromic article. Details of the spectacle lens included in the eyeglasses are as described above. By providing such a spectacle lens, for example, the eyeglasses can exhibit an anti-glare effect like sunglasses when the photochromic compound is colored upon receiving sunlight outdoors, and the photochromic compound can fade upon returning indoors, and thus the transmittance can be recovered. For the eyeglasses, a known technique can be applied to the configuration of the frame or the like.

### [Examples]

Hereinafter, the present invention will be described in more detail with reference to examples. However, the present invention is not limited to embodiments shown in examples.

### [Synthesis of Exemplary Compound 1]

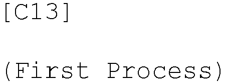

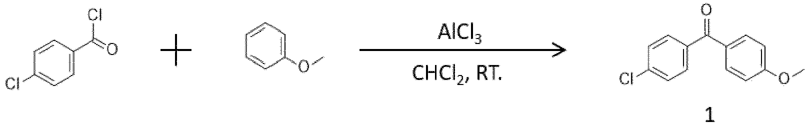

Under an argon atmosphere, a dichloromethane solution (400 ml) containing 4-chlorobenzoyl chloride (35.6 g, 200 mmol) was ice-cooled, and aluminum chloride (27.1 g, 200 mmol) was added. Subsequently, a dichloromethane solution (140 ml) containing anisole (20.0 g, 190 mmol) was added dropwise over 15 minutes and the mixture was stirred at room temperature for 2 hours. The reaction solution was poured into 1 N hydrochloric acid (200 ml) and ice, stirred, and liquid-separated. The organic layer was washed with a saturated sodium bicarbonate aqueous solution and a saturated saline, and then dried with sodium sulfate. Filtration and concentration were performed to obtain Compound 1 (47.9 g) as a colorless solid.

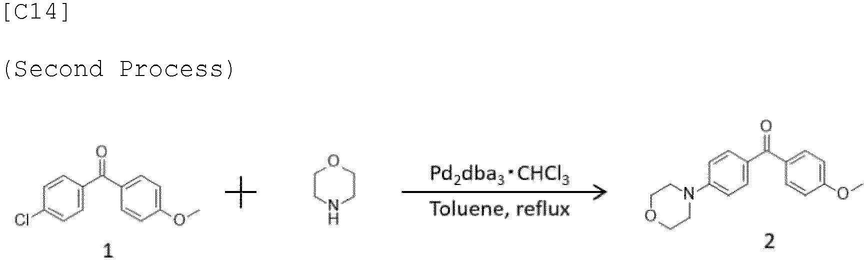

Under an argon atmosphere, a toluene solution (81 ml) containing Compound 1 (20.0 g, 81.0 mmol), Pd₂dba₃·CHCl₃ (0.84 g, 0.81 mmol), XPhos (1.16 g, 2.43 mmol), sodium tert-butoxy (10.9 g, 114 mmol), and morpholine (10.6 g, 122 mmol) was stirred at an outside temperature of 105°C for 2 hours. After cooling, the mixture was filtered through celite, and unnecessary substances were washed with chloroform (200 ml). The filtrate was concentrated to obtain Compound 2 (27.1 g) as a lighter yellow solid.

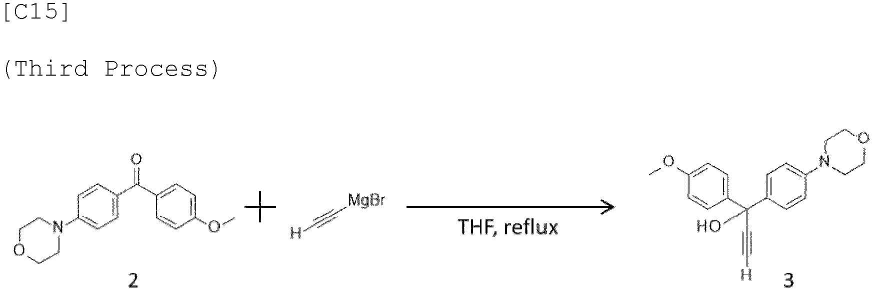

Under an argon atmosphere, a tetrahydrofuran solution (420 ml) containing Compound 2 (25.0 g, 84.0 mmol) was ice-cooled, and ethynylmagnesium bromide (250 ml, 0.5 M, 126 mmol) was added dropwise over 20 minutes. The mixture was stirred at an outside temperature of 65°C for 2 hours. After the reaction solution was cooled, quenching with a saturated aqueous sodium chloride solution (300 ml) was performed and extraction with ethyl acetate (200 ml×2 times) was performed. The organic layer was washed with a saturated saline and then dried with sodium sulfate. After filtration and concentration, the residue was purified through silica gel column chromatography (SiO₂: 400 g, heptane/ethyl acetate=98/2 to 95/5) to obtain Compound 3 (11.2 g) as a lighter yellow solid.

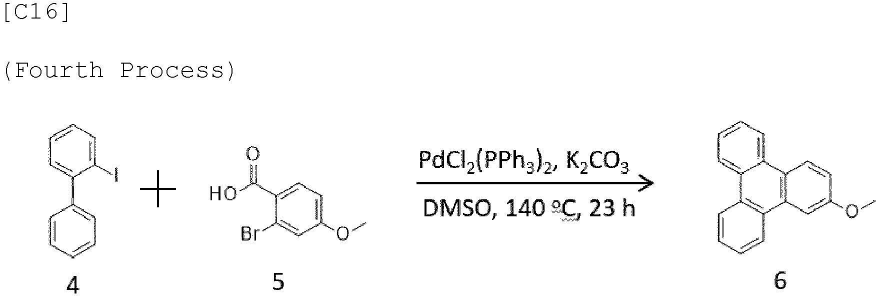

Under an argon atmosphere, a DMSO solution (90 mL) containing Compound 4 (5.00 g, 17.9 mmol), Compound 5 (4.54 g, 19.6 mol), potassium carbonate (7.40 g, 53.6 mmol), and dichlorobis(triphenylphosphine)palladium (0.63 g, 0.89 mmol) was stirred at 140°C for 23 hours. After cooling, water (200 mL) was added, extraction with ethyl acetate/heptane (80 mL/40 mLx2) was performed, and the organic layer was washed with water and a saturated saline. After drying with sodium sulfate, filtration and concentration were performed. The obtained residue was purified through column chromatography (SiO₂: 100 g, heptane/ethyl acetate=98/2 to 95/5) to obtain Compound 6 (3.8 g).

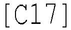

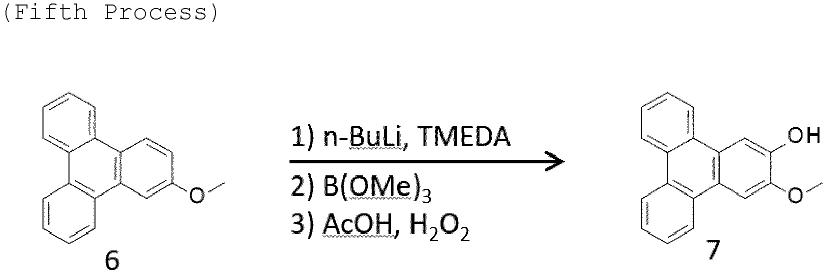

Under an argon atmosphere, a THF solution (50 mL) containing Compound 6 (2.68 g, 11.6 mol) was cooled at an outside temperature of -78°C, and TMEDA (2.63 mL, 17.4 mmol) and n-butyl lithium (1.59 M, 11.0 mL, 17.4 mmol) were added thereto.

Heating was performed to an outside temperature of 0°C over 1 hour, and the mixture was directly stirred for 2 hours. Trimethyl borate (2.30 mL, 20.3 mmol) was added and the mixture was stirred at room temperature for 4 hours. Under ice-cooling, acetic acid (1.33 mL, 23.2 mmol) and a hydrogen peroxide solution (30%, 2.40 mL, 27.2 mmol) were added thereto and the mixture was stirred at room temperature overnight. An ammonium chloride aqueous solution was added thereto, extraction with ethyl acetate (50 mLx2) was performed, and the organic layer was washed with a sodium thiosulfate aqueous solution and a saturated saline and dried with sodium sulfate. Filtration and concentration were performed, and the residue was purified through column chromatography (SiO₂: 400 g, heptane/ethyl acetate=95/5 to 90/10) to obtain Compound 7(1.65 g).

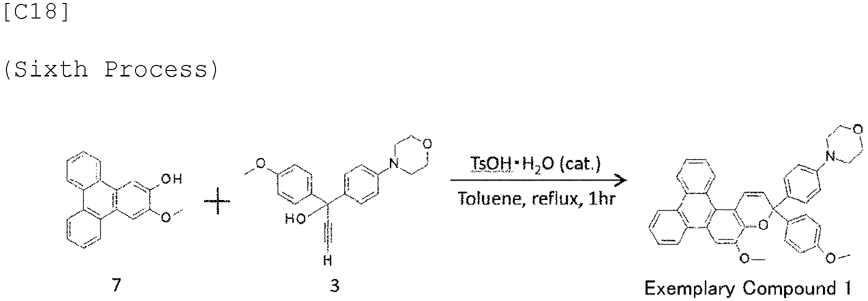

Under an argon atmosphere, p-toluene sulfonic acid monohydrate (0.11 g, 0.58 mmol) was added to a toluene suspension (35 mL) containing Compound 7 (0.80 g, 2.92 mol) and Compound 3 (1.60 g, 4.37 mmol), and the mixture was stirred at an outside temperature of 120°C for 2 hours. After cooling, the mixture was filtered through celite, washed with ethyl acetate and concentrated. The obtained residue was purified through column chromatography (SiO₂: 400 g, heptane/ethyl acetate=95/5 to 60/40). The obtained solid was suspended and washed using diisopropyl ether (50 mL) to obtain Exemplary Compound 1.

The obtained products were analyzed by the following method.

### (1) Purity Analysis

The purity was calculated from the area ratio of high performance liquid chromatography (HPLC). LC-2040C (commercially available from Shimadzu Corporation) (YMC-Triart C18 100x2.1 mm, column temperature 40°C, PDA detector) was used as an HPLC device. For the mobile phase, under conditions of acetonitrile/0.1% TFA H₂O=5/95-95/5 (7 min), 95/5 (3 min), the flow rate was 0.4 ml/min.

### (2) Structure Identification

An LC-MS method was used for mass spectrometry. As an LC-MS device, ACQUITY UPLC H-Class system (commercially available from Nihon Waters K.K.) (liquid chromatograph part), SQD2 (mass spectrometry part), and ACQUITY UPLC BEH C18 (ϕ2.1×50 mm (column part) were used. As a result of mass spectrometry, the measured value was shown in Table 1 for the calculated value of the exact mass shown in Table 1.
CHN elemental analysis was used for elemental analysis. Series II 2400 (commercially available from PerkinElmer Co., Ltd.) was used as a CHN elemental analysis device. Regarding the calculated values shown in Table 1, the measured values were the values shown Table 1.

ECS-400 (commercially available from JEOL Ltd.) was used for proton nuclear magnetic resonance (¹H-NMR). Deuterated chloroform was used as a measurement solvent. Fig. 1 shows the obtained NMR chart.

It was confirmed based on the above analysis results that desired compounds were produced comprehensively.

### [Synthesis of Exemplary Compounds 2 to 5]

Exemplary Compounds 2 to 5 were obtained by performing the same operation using the same synthesis method except that compounds shown in Table 1 were used in place of Compound 7 and Compound 3 (synthetic intermediate). Table 1 shows respective molecular structures and the analysis results of the obtained compounds.

### [Synthesis of Comparative Compound 1]

Regarding the synthesis method, Comparative Compound 1 was synthesized according to the method described in the document described above. Table 1 shows the analysis results of the obtained Comparative Example Compound 1.

### [Evaluation Method]

### <Evaluation of Absorption Property Before and After UV emission>

Respective compounds of examples and a comparative example were dissolved in chloroform containing no stabilizer to prepare a chloroform solution of the compound.

A 1 cm square quartz spectroscopic cell containing the prepared solution was covered, and the absorbance was measured using an ultraviolet and visible spectrophotometer (UV-1900i, commercially available from Shimadzu Corporation, measurement wavelength: 800 to 250 nm, wavelength increments of 2 nm, high speed mode). The absorbance was measured at room temperature (20 to 25°C). In addition, the solution was once taken out from the spectrophotometer and ultraviolet rays were emitted for 15 seconds using UV-LED (commercially available from Hamamatsu Photonics K.K.) (a combination of LIGHTNINGCURE LC-L1V5 and L14310-120, an output of 70%) as an ultraviolet light source. During UV emission, the solution was stirred with a small stirrer. Within 10 seconds after UV emission was completed, the sample was set again in the UV-visible spectrophotometer, spectroscopic measurement was performed under the same conditions, and the first absorption wavelength was determined.

The molar extinction coefficient ε (M⁻¹ cm⁻¹) at a wavelength of 380 nm before UV emission was read. The range corresponds to the absorption base of the uncolored component, and the molar extinction coefficient at 380 nm can be used as an index of coloring of the uncolored component. ε was preferably less than 5,000 M⁻¹ cm⁻¹ because a feeling of coloring was weak, and more preferably less than 3,000 M⁻¹ cm⁻¹. Table 1 shows the values of the read molar extinction coefficient at 380 nm.

Since the colored component was formed after UV emission, new absorption peaks appeared in the visible light range (380 nm to 800 nm). Among absorption peaks appearing in the visible range, the wavelength of the peak of the absorption intensity observed at the longest wavelength was read, and shown as "first absorption wavelength" in Table 1.

As an example, absorption spectrums of Exemplary Compound 1 and Comparative Compound 1 before and after UV emission are shown in Fig. 2.

**[Table 1]**

| | Desired Product | Synthetic Intermediate | | HPLC Purity (%) | Calculated value of exact mass | Mass spectrometry measured value | CHN composition calculated value (%) | CHN elemental analysis measured value (%) | Molar extinction coefficient at 380 nm before UV emission (M⁻¹cm⁻¹) | First absorption wavelength after UV emission (nm) |
|---|---|---|---|---|---|---|---|---|---|---|
| Exemplary Compound 1 | 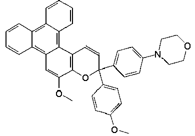 | 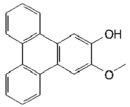 | 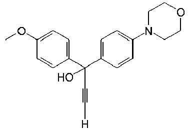 | 98 | 579.241 | 579.6 | C: 80.8% | C: 81.2% | 1793 | 480 |
| | | | | | | | H: 5.7% | H: 6.0% | | |
| | | | | | | | N: 2.4% | N: 2.2% | | |
| Exemplary Compound 2 | 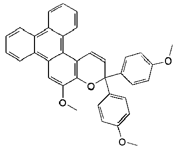 | 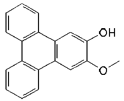 | 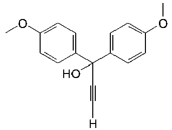 | 98 | 524.199 | 524.3 | C: 82.4% | C: 83.0% | 1852 | 492 |
| | | | | | | | H: 5.4% | H: 5.5% | | |
| Exemplary Compound 3 | 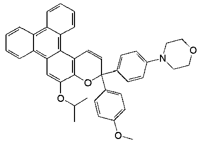 | 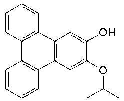 | 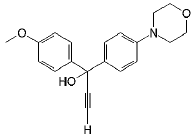 | 97 | 607.272 | 606.9 | C: 81.0% | C: 81.5% | 1944 | 534 |
| | | | | | | | H: 6.1% | H: 6.0% | | |
| | | | | | | | N: 2.3% | N: 2.2% | | |
| Exemplary Compound 4 | 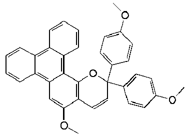 | 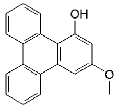 | 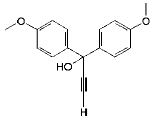 | 98 | 524.199 | 524.2 | C: 82.4% | C: 82.8% | 2205 | 574 |
| | | | | | | | H: 5.4% | H: 6.2% | | |
| Exemplary Compound 5 | 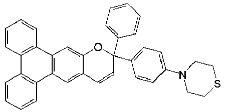 | 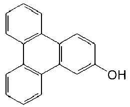 | 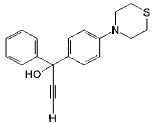 | 98 | 535.197 | 535.4 | C: 83.0% | C: 83.0% | 2480 | 596 |
| | | | | | | | H: 5.5% | H: 5.4% | | |
| | | | | | | | N: 2.6% | N: 3.0% | | |
| Comparative Compound 1 | 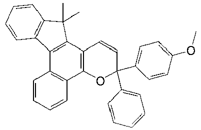 | | | 98 | 480.209 | 480.3 | C: 87.5% | C: 87.6% | 6108 | 542 |
| | | | | | | | H: 5.9% | H: 6.0% | | |

For Exemplary Compounds 1 to 5, new absorption peaks appeared in the visible light range after UV emission, and it was confirmed that these compounds were compounds exhibiting photochromic properties. Accordingly, the compounds exhibiting photochromic properties could be used for producing various photochromic articles such as spectacle lenses.

In addition, based on the results shown in Table 1, it was confirmed that Exemplary Compounds 1 to 5 absorbed less visible light having a short wavelength in the uncolored component than Comparative Example Compound 1 having no triphenylene framework.

### [Production and Evaluation of Spectacle Lens]

### <Preparation of Photochromic Composition (Polymerizable Composition)>

In a plastic container, with respect to a total amount of 100 parts by mass of (meth)acrylates, 68 parts by mass of polyethylene glycol diacrylate, 12 parts by mass of trimethylolpropane trimethacrylate, and 20 parts by mass of neopentyl glycol dimethacrylate were mixed to prepare a (meth)acrylate mixture. 7 parts by mass of Exemplary Compound 1 was mixed with respect to 100 parts by mass of the (meth)acrylate mixture. In addition, a photopolymerization initiator (phenyl bis(2,4,6-trimethylbenzoyl)phosphine oxide), an antioxidant [bis(3-tert-butyl-4-hydroxy-5-methylphenyl)propionic acid)] [ethylene bis(oxyethylene)] and a light stabilizer (bis(1,2,2,6,6-pentamethyl-4-piperidyl) sebacate) were mixed and sufficiently stirred and a silane coupling agent (γ-methacryloxypropyltrimethoxysilane) was then added dropwise with stirring. Then, defoaming was performed using an automatic revolution type stirring and defoaming device.

A photochromic composition was prepared by the above method.

### <Formation of Primer Layer>

A plastic lens substrate (commercially available from HOYA product name EYAS: a center thickness of 2.5 mm, a diameter of 75 mm, and a spherical lens power of -4.00) was immersed in a sodium hydroxide aqueous solution having a concentration of 10 mass% (a liquid temperature of 60°C) for 5 minutes, washed with an alkali and additionally washed with pure water and dried. Then, a water-based polyurethane resin liquid (polycarbonate polyol-based polyurethane emulsion, a viscosity of 100 cPs, and a solid content concentration of 38 mass%) was applied to the convex surface of the plastic lens substrate in an environment of room temperature and a relative humidity of 40 to 60% using a spin coater MS-B150 (commercially available from Mikasa Corporation) at a rotational speed of 1,500 rpm for 1 minute according to a spin coating method and then dried naturally for 15 minutes, and thereby a primer layer having a thickness of 5.5 um was formed.

### <Formation of Photochromic Layer>

The photochromic composition prepared above was added dropwise to the primer layer, and applied by a spin coating method using a program in which the rotational speed was changed in a slope mode from a rotational speed of 500 rpm to 1,500 rpm over 1 minute, and rotation was additionally performed at 1,500 rpm for 5 seconds using MS-B150 (commercially available from Mikasa Corporation). Then, ultraviolet rays (with a dominant wavelength of 405 nm) were emitted to the photochromic composition applied on the primer layer formed on the plastic lens substrate in a nitrogen atmosphere (with an oxygen concentration of 500 ppm or less) for 40 seconds, and the composition was cured to form a photochromic layer. The thickness of the formed photochromic layer was 45 um.

Accordingly, a photochromic article (spectacle lens) was produced.

### <Evaluation of Coloring Concentration>

The luminous reflectance was obtained by the following method according to JIS T 7333: 2005.

Light was emitted to the convex surface of the spectacle lens using a xenon lamp as a light source through an air mass filter for 15 minutes, and the photochromic layer was colored. Light emission was performed so that the irradiance and irradiance tolerance were values shown in Table 2 as specified in JIS T 7333: 2005. The transmittance during the coloring was measured with a spectrophotometer (commercially available from Otsuka Electronics Co., Ltd.).

**[Table 2]**

| Wavelength range(nm) | Irradiance (W/m²) | Irradiance tolerance (W/m²) |
|---|---|---|
| 300∼340 | <2.5 | |
| 340∼380 | 5.6 | ±1.5 |
| 380∼420 | 12 | ±3.0 |
| 420∼460 | 12 | ±3.0 |
| 460∼500 | 26 | ±2.6 |

### <Evaluation of Fade Rate>

The fade rate was evaluated by the following method.

The transmittance (measurement wavelength: 550 nm) of the spectacle lens before light emission (uncolored state) was measured with a spectrophotometer (commercially available from Otsuka Electronics Co., Ltd.). The transmittance measured here is called an "initial transmittance".

Light was emitted to each spectacle lens using a xenon lamp as a light source through an air mass filter for 15 minutes, and the photochromic layer was colored. Light emission was performed so that the irradiance and irradiance tolerance were values shown in Table 2 as specified in JIS T 7333: 2005. The transmittance during the coloring was measured in the same manner as the initial transmittance. The transmittance measured here is called a "transmittance during coloring".

Then, the time required for the transmittance to reach [(initial transmittance-transmittance during coloring)/2] from the time when light emission was stopped was measured.

The spectacle lens containing Exemplary Compound 1 had a luminous transmittance T% of 42% during coloring and had a half-life time of 4.5 minutes.

Based on the above results, it was confirmed that the above spectacle lens was a spectacle lens (photochromic lens) exhibiting photochromic properties in which the luminous transmittance before and after UV emission changed and the state was returned to the original state over time when emission of ultraviolet rays was stopped.

Finally, the above aspects are summarized.

According to one aspect, there are provided photochromic compounds represented by General Formula 1, photochromic compounds represented by General Formula 2 and photochromic compounds represented by General Formula 3.

In one aspect, the substituent that can be represented by any of R¹⁰⁰ to R¹¹¹, R²⁰⁰ to R²¹¹, R³⁰⁰ to R³¹¹ and X¹ to X⁶ in General Formulae 1 to 3 may be
a substituent R^{m} selected from the group consisting of a hydroxy group, linear or branched alkyl groups having 1 to 18 carbon atoms, monocyclic or multicyclic cycloaliphatic alkyl groups having 5 to 18 carbon atoms, linear or branched alkoxy groups having 1 to 24 constituent atoms, non-aromatic cyclic substituents having 1 to 24 constituent atoms, linear or branched perfluoroalkyl groups having 1 to 18 carbon atoms, linear or branched perfluoroalkoxy group, linear or branched alkylsulfide groups having 1 to 24 constituent atoms, an aryl group, aryloxy group, arylsulfide group, heteroaryl group, amino group, monoalkylamino group, dialkylamino group, monoarylamino group, diarylamino group, cyclic amino group, ethynyl group, mercapto group, silyl group, sulfonic acid group, alkylsulfonyl group, formyl group, carboxy group, and cyano group and halogen atoms; or
a substituent in which R^{m} is additionally substituted with one or more of the same or different R^{m}'s.

According to one aspect, there is provided a photochromic composition containing one or more photochromic compounds selected from the group consisting of photochromic compounds represented by General Formula 1, photochromic compounds represented by General Formula 2 and photochromic compounds represented by General Formula 3.

In one aspect, the photochromic composition may further include a polymerizable compound.

According to one aspect, there is provided a photochromic article including a cured product obtained by curing the photochromic composition.

In one aspect, the photochromic article may include a substrate and a photochromic layer which is the cured product.

In one aspect, the photochromic article may be a spectacle lens.

In one aspect, the photochromic article may be a goggle lens.

In one aspect, the photochromic article may be a visor part of a sun visor.

In one aspect, the photochromic article may be a shield member of a helmet.

According to one aspect, there is provided eyeglasses including the spectacle lens.

Two or more of various aspects and various forms described in this specification can be combined in arbitrary combinations.

The embodiments disclosed herein are only examples in all respects and should not be considered as restrictive. The scope of the present invention is not limited to the above description, but is defined by the scope of claims, and is intended to encompass equivalents to the scope of claims and all modifications within the scope of the claims.

### [Industrial Applicability]

One aspect of the present invention is beneficial in the technical fields of eyeglasses, goggles, sun visors, helmets and the like.

## Claims

1. A photochromic compound represented by the following General Formula 1: 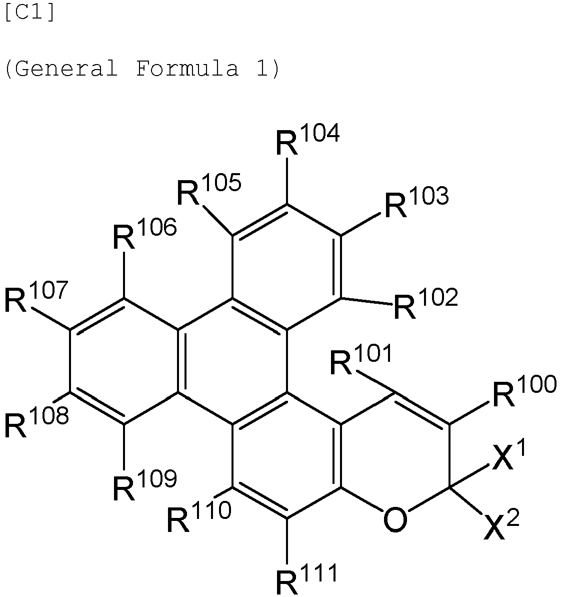 (in General Formula 1, R¹⁰⁰ to R¹¹¹, and X¹ and X² each independently represent a hydrogen atom or a substituent, and two or more substituents may be bonded to form a ring structure).

2. A photochromic compound represented by the following General Formula 2: 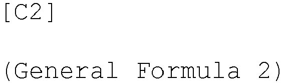 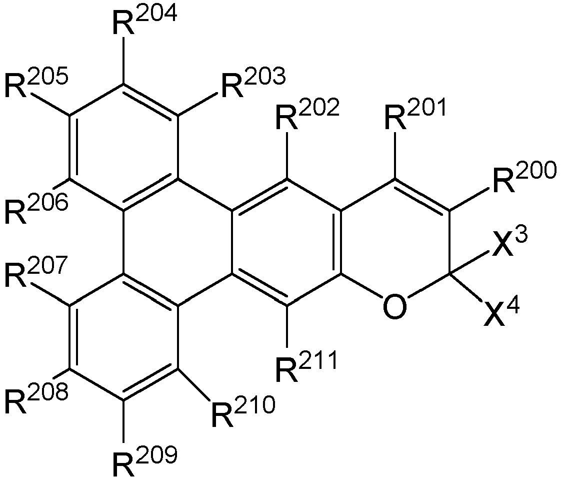 (in General Formula 2, R²⁰⁰ to R²¹¹, and X³ and X⁴ each independently represent a hydrogen atom or a substituent, and two or more substituents may be bonded to form a ring structure).

3. A photochromic compound represented by the following General Formula 3: 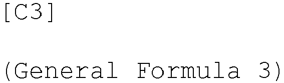 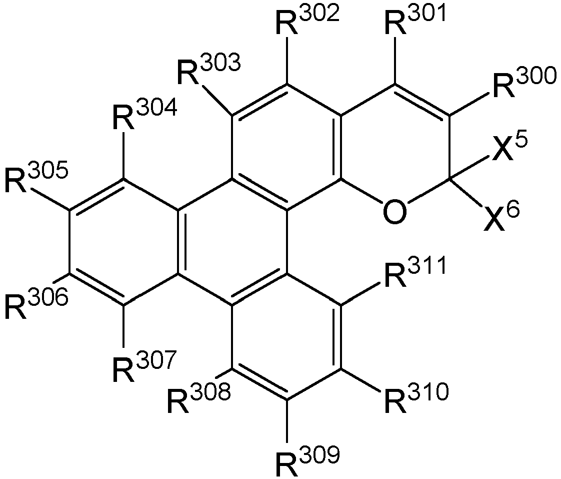 (in General Formula 3, R³⁰⁰ to R³¹¹, and X⁵ and X⁶ each independently represent a hydrogen atom or a substituent, and two or more substituents may be bonded to form a ring structure).

4. The photochromic compound according to any one of claims 1 to 3,
wherein the substituent is
a substituent R^{m} selected from the group consisting of a hydroxy group, linear or branched alkyl groups having 1 to 18 carbon atoms, monocyclic or multicyclic cycloaliphatic alkyl groups having 5 to 18 carbon atoms, linear or branched alkoxy groups having 1 to 24 constituent atoms, non-aromatic cyclic substituents having 1 to 24 constituent atoms, linear or branched perfluoroalkyl groups having 1 to 18 carbon atoms, linear or branched perfluoroalkoxy group, linear or branched alkylsulfide groups having 1 to 24 constituent atoms, an aryl group, aryloxy group, arylsulfide group, heteroaryl group, amino group, monoalkylamino group, dialkylamino group, monoarylamino group, diarylamino group, cyclic amino group, ethynyl group, mercapto group, silyl group, sulfonic acid group, alkylsulfonyl group, formyl group, carboxy group, and cyano group and halogen atoms; or
a substituent in which R^{m} is additionally substituted with one or more of the same or different R^{m}'s.

5. A photochromic composition comprising one or more photochromic compounds selected from the group consisting of the photochromic compound according to claim 1, the photochromic compound according to claim 2 and the photochromic compound according to claim 3.

6. The photochromic composition according to claim 5,
wherein the substituent is
a substituent R^{m} selected from the group consisting of a hydroxy group, linear or branched alkyl groups having 1 to 18 carbon atoms, monocyclic or multicyclic cycloaliphatic alkyl groups having 5 to 18 carbon atoms, linear or branched alkoxy groups having 1 to 24 constituent atoms, non-aromatic cyclic substituents having 1 to 24 constituent atoms, linear or branched perfluoroalkyl groups having 1 to 18 carbon atoms, linear or branched perfluoroalkoxy group, linear or branched alkylsulfide groups having 1 to 24 constituent atoms, an aryl group, aryloxy group, arylsulfide group, heteroaryl group, amino group, monoalkylamino group, dialkylamino group, monoarylamino group, diarylamino group, cyclic amino group, ethynyl group, mercapto group, silyl group, sulfonic acid group, alkylsulfonyl group, formyl group, carboxy group, and cyano group and halogen atoms; or
a substituent in which R^{m} is additionally substituted with one or more of the same or different R^{m}'s.

7. The photochromic composition according to claim 5 or 6, further comprising a polymerizable compound.

8. A photochromic article comprising a cured product obtained by curing the photochromic composition according to claim 7.

9. The photochromic article according to claim 8, comprising a substrate and a photochromic layer which is the cured product.

10. The photochromic article according to claim 8 or 9, which is a spectacle lens.

11. The photochromic article according to claim 8 or 9, which is a goggle lens.

12. The photochromic article according to claim 8 or 9, which is a visor part of a sun visor.

13. The photochromic article according to claim 8 or 9, which is a shield member of a helmet.

14. Eyeglasses comprising the spectacle lens according to claim 10.
